(19) Europäisches Patentamt
European Patent Office
Office européen des brevets

(11) EP 0 597 289 B1

(12) EUROPEAN PATENT SPECIFICATION

(45) Date of publication and mention of the grant of the patent:
**12.03.1997 Bulletin 1997/11**

(51) Int Cl.[6]: **G03C 1/04**, G03C 1/047, G03C 1/06, G03C 1/30, G03C 1/74

(21) Application number: **93116962.7**

(22) Date of filing: **05.11.1993**

# (54) Photographic composition containing a thickening agent

Photographische Zusammensetzung enthaltend einen Verdicker

Composition photographique comprenant un épaississeur

(84) Designated Contracting States:
**BE CH DE FR GB IT LI NL**

(30) Priority: **12.11.1992 US 975238**

(43) Date of publication of application:
**18.05.1994 Bulletin 1994/20**

(73) Proprietor: **EASTMAN KODAK COMPANY**
**Rochester, New York 14650-2201 (US)**

(72) Inventors:
- **Ross, Robert John,**
  **c/o EASTMAN KODAK COMPANY**
  **Rochester, New York 14650-2201 (US)**
- **Bagchi, Pranab,**
  **c/o EASTMAN KODAK COMPANY**
  **Rochester, New York 14650-2201 (US)**
- **Connelly, Richard Wayne,**
  **c/o EASTMAN KODAK COMPANY**
  **Rochester, New York 14650-2201 (US)**

(74) Representative:
**Wibbelmann, Jobst, Dr., Dipl.-Chem. et al**
**Wuesthoff & Wuesthoff,**
**Patent- und Rechtsanwälte,**
**Schweigerstrasse 2**
**81541 München (DE)**

(56) References cited:
**EP-A- 0 358 073**
**EP-A- 0 367 572**
**WO-A-91/14967**
**FR-A- 2 150 505**
**US-A- 3 926 869**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 597 289 B1

## Description

### Technical Field

This invention relates to a photographic composition containing a thickening agent, to a process for preparing said composition, to a photographic element prepared therefrom, to a method of preparing said photographic element, and to a novel compound useful as a thickening agent in photographic compositions.

### Prior Art

Photographic elements typically comprise a plurality of photosensitive layers and non-photosensitive layers, such as, interlayers, anti-halation layers, protective layers and the like. The layers generally are coated from fluid solutions containing a hydrophilic colloid, such as gelatin. Usually, coating takes place at temperatures between about 35 to about 50°C. The fluid solutions are frequently referred to as "melts" or "coating melts".

### Assessment of the Art

Generally, in the photographic art there are two primary methods of coating photographic compositions. One is the bead coating process. U.S. Patent 2,761,417 to Russell et al., U.S. Patent 2,681,294 to Beguin, and U.S. Patent 4,525,392 to Ishizaki illustrate simultaneously applying multiple layers of photographic compositions by bead coating processes, and apparatus for practicing those processes.

The second primary method is the curtain coating process. U.S. Patent 3,632,374 to Greiller, and U.S. Patent 4,569,863 to Koepke et al., illustrate apparatus and processes for curtain coating.

It is well known that adjustment and control of viscosities of typical photographic coating melts of individual layers can improve uniformity of layer thickness of the coated product. It is also known that layer viscosities outside optimum ranges may cause undesired variations in layer thicknesses during flow on the slides of the coating hopper or on a non-horizontal web path after coating.

Coating melts with low gelatin concentrations, below about 4% gelatin, have relatively low viscosities and as a result are particularly difficult to coat in multilayer slide hopper coating machines without undesired variation in layer thickness. Usually, in multilayer curtain coating devices even higher viscosities are desirable for producing uniform coatings.

There are certain advantages, however, in using low gelatin-containing melts. In general, the lower the gelatin content, the thinner the resulting coating. Thinner layers in photographic coatings provide the advantages of (1) sharper images, and (2) faster processing films.

It has been proposed to incorporate polymeric materials into gelatin-containing photographic coating compositions to increase the viscosity thereof. See for example U.S. Patents 3,861,924 to Mackey et al and 3,926,869 to Horie et al and Soviet patent publication SU 857,917 to Korneva. However, the use of polymeric materials can add to the cost of the product as polymers with sufficient purity and specific molecular weight or molecular weight distribution for photographic use are expensive to manufacture.

Therefore, there is a need to control the viscosity of gelatin-containing photographic melts in a cost effective manner and in particular to formulate high viscosity, low gelatin-containing photographic melts that are suitable for producing relatively thin, uniform multilayer product coatings, especially in multiple layer slide hopper or curtain coating machines.

### Disclosure of the Invention

One aspect of the invention concerns the use of a compound of the formula:

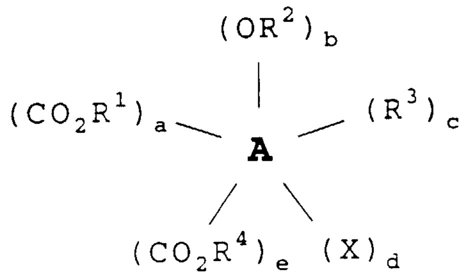

Formula A

wherein:

A is an aromatic or heteroaromatic group;

X is $SO_3M$ or $CO_2M$, where M is H or an alkali metal, ammonium or pyridinium ion;

each $R^1$, $R^2$, and $R^3$ is independently an alkyl or substituted alkyl group, each alkyl group being straight or branched chain and having 1 to 30 carbon atoms;

$R^4$ is H or an alkyl or substituted alkyl group, each alkyl group being straight or branched chain and having 1 to 30 carbon atoms;

each of a, b, c and e is independently 0, 1, 2 or 3, with the proviso the sum of a + b + c + e equals at least 1; and

d is 1, 2, or 3;

with the further proviso that the sum of a + b + c + e is at least 2 when A is phenyl, X is $SO_3M$, d is 1 and any one of a, c, or e is 1;

as a thickening agent in a photographic composition comprising an aqueous medium containing a hydrophilic colloid.

Further aspects of the invention are set forth in the dependent claims.

A still further aspect of this invention is a novel compound of the formula:

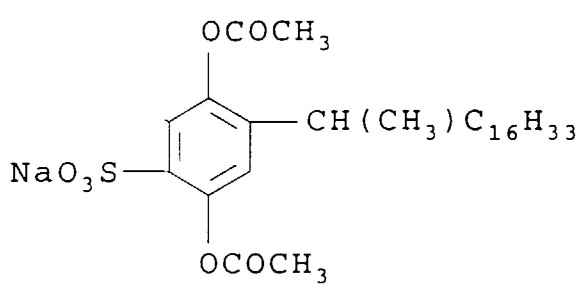

## Advantageous Effect of the Invention

This invention provides a thickening agent for photographic compositions which permit melts of the compositon to be applied as a substantially uniform layer to a support using conventional coating equipment. The thickening agent increases the viscosity of coating melts of the composition permitting control of the viscosity by adding an appropriate amount of the thickening agent. If the gelatin content of the photographic composition is low, a comparatively thin uniform layer can be achieved, thus providing sharper images and faster processing times.

## Brief Description of the Figures

FIG. 1. illustrates attachment of multiple gelatin molecules to a compound used as a thickening agent in accordance with this invention.

FIG. 2 illustrates attachment of multiple units of Fig. 1 to each other.

FIG. 3a and FIG. 3b illustrate attachment of multiple thickening agent molecules to a gelatin molecule.

FIG. 4 depicts rheograms of melts of compositions of this invention as set forth in the examples below.

FIG. 5 is a graph setting forth the viscosity in the melt of compositions of this invention as set forth in the examples below.

A Detailed Account of at least one way of carrying out the Invention

The photographic composition of this invention comprises an aqueous medium containing a hydrophilic colloid, preferably gelatin. The composition may contain gelatin of any kind such as lime processed ossein, acid processed ossein, pig skin gelatin or derivatized gelatins as described by Bagchi (U.S. 4,920,004; U.S. 5,026,632; U.S. 5,055,379; and U.S. 5,013,640).

The composition of this invention may contain conventional photographic agents such as silver halide emulsions, coupler dispersions, dispersions of other photographic agents, synthetic polymers, and soluble chemicals such as oxidized developer scavengers, dyes, optical brighteners, masking couplers, surfactants, and the like. A summary of photographic addenda which can be incorporated into the aqueous media can be found in Research Disclosure 308,119 dated December 1989, the disclosure of which is incorporated herein by reference.

The amount of hydrophilic colloid present in the composition of this invention is about 3 to about 20% by weight, based on the weight of the composition. As mentioned above, the invention has particular utility preparing thickened photographic melts in which the hydrophilic colloid content is less than about 4%. Percentages reported in this patent application are by weight, based on the total weight of the composition, unless otherwise specified. The hydrophilic colloid is preferably gelatin.

The thickening agent utilized in accordance with this invention is an organic chemical compound that is photographically inert and has large hydrophobic groups and one or more anionic groups such as $-SO_3^-$ (i.e., sulfonate) groups, sulfate groups or carboxy groups in the molecule. Such compounds are generally soluble to the extent of at least about 5%, by weight, in water, gelatin-containing aqeuous media, or a solution of water containing about 5 to about 20%, by weight, of a water miscible auxiliary solvent such as methanol, ethanol, propanol, isopropanol, acetone, methyl ethyl ketone, ethyl acetate, or the like.

The thickening agent utilized in this invention can be represented by the general structural formula:

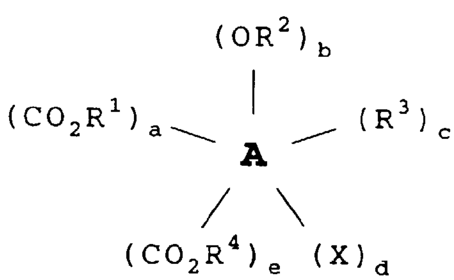

Formula A

wherein:

A is an aromatic or heteroaromatic group;
X is $SO_3M$ or $CO_2M$
M is H or an alkali metal, ammonium or pyridinium ion; each $R^1$, $R^2$, and $R^3$ is independently an alkyl or substituted alkyl group, each alkyl group being straight or branched chain and having 1 to 30 carbon atoms;
$R^4$ is H or an alkyl or substituted alkyl group, each alkyl group being straight or branched chain and having 1 to 30 carbon atoms;
each of a, b, c and e is independently 0, 1, 2 or 3, with the proviso that the sum of a + b + c + e equals at least 1; and
d is 1, 2, or 3;

with the further proviso that when A is phenyl, X is $SO_3M$, d is 1 and any one of a, c, or e is 1, the sum of a + b + c + e is at least 2.

The use of anionic compounds containing a hydrophobic group as surfactants in photographic compositions is known. See for example U.S. Patents 3,860,425 to Ono et al and 3,912,517 to Van Poucke et al.

Preferred compounds of Formula A are those in which A is phenyl, X is $SO_3M$, M is H, or a sodium, potassium,

pyridium, or ammonium ion, $R^3$ is a straight or branched chain alkyl group having 1 to 30 carbon atoms, $R^4$ is a straight or branched chain alkyl group having 1 to 30 carbon atoms, d is 1, 2 or 3, c is 1, e is 2 or 3 and a and b are each 0. Particularly preferred is the compound of structure No. 1 set forth below.

The compounds used as thickening agents in accordance with this invention are generally known compounds and can be prepared by known methods. The compound of structure No. 1, below, is novel and a method for preparing this compound is set forth in the preparative example below.

Examples of compounds of compounds useful as thickening agents in accordance with this invention include, but are not limited to, compounds of the following structures:

No. 1:

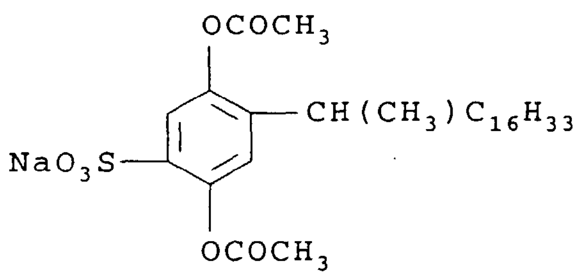

No. 2:

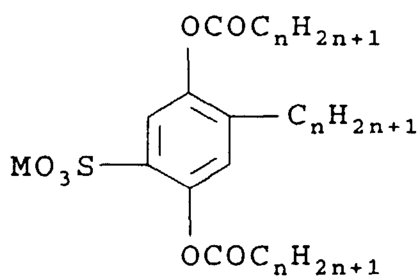

No. 3:

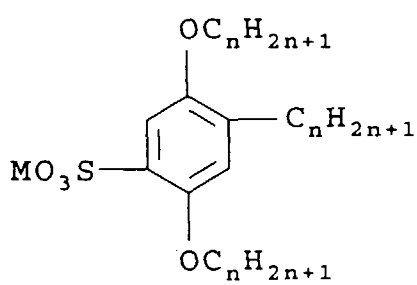

No. 4:

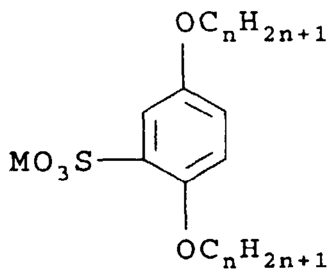

No. 5:

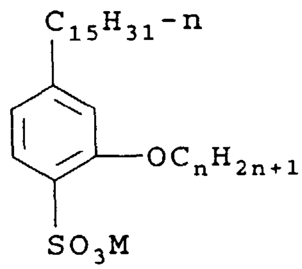

No. 6:

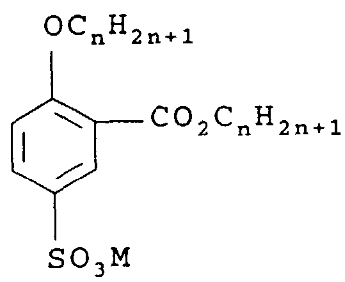

No. 7:

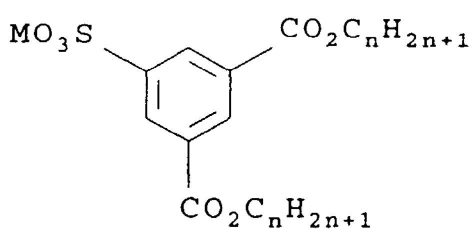

No. 8:

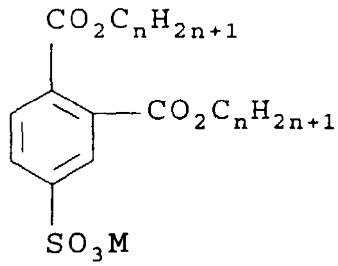

No. 9:

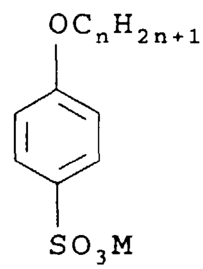

No. 10:

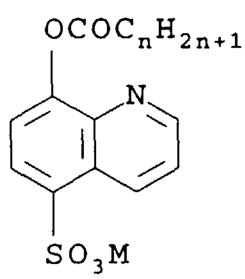

No. 11:

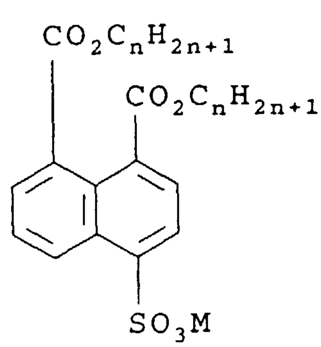

No. 12:

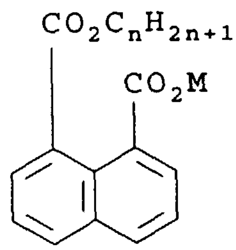

No. 13:

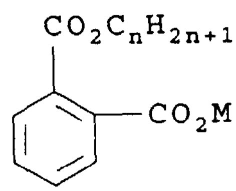

No. 14:

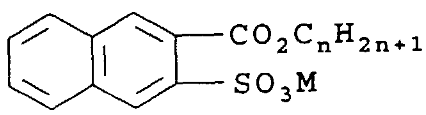

No. 15:

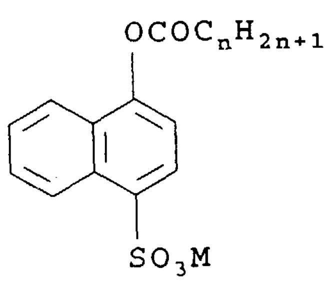

No. 16:

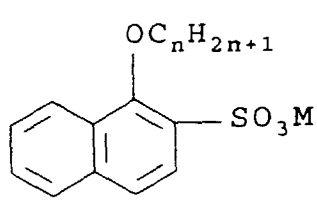

No. 17:

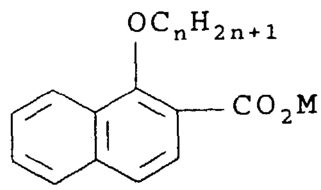

No. 18:

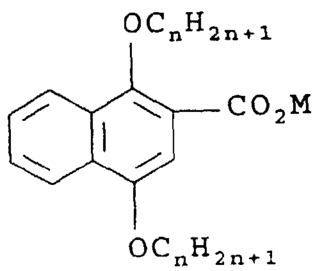

No. 19:

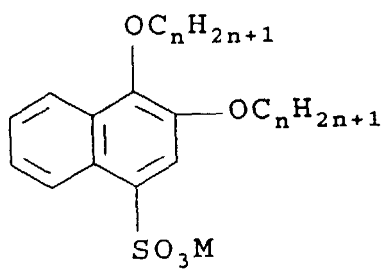

No. 20:

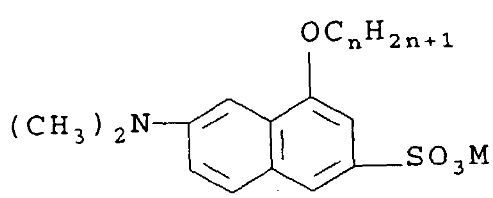

No. 21:

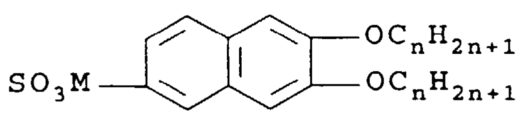

No. 22:

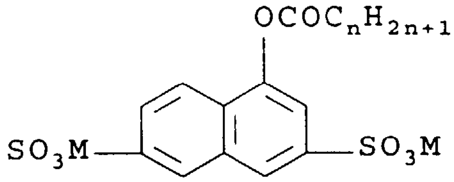

No. 23:

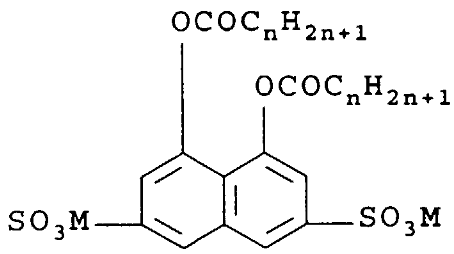

No. 24:

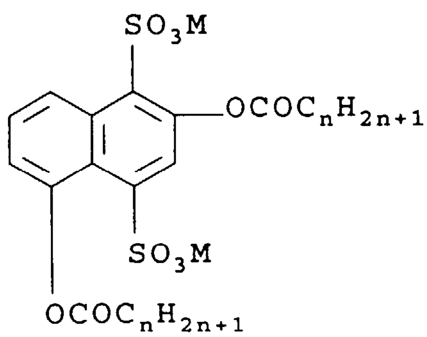

No.25:

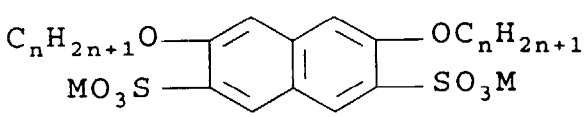

No. 26:

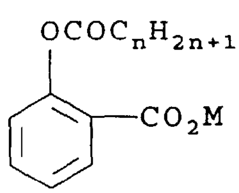

No. 27:

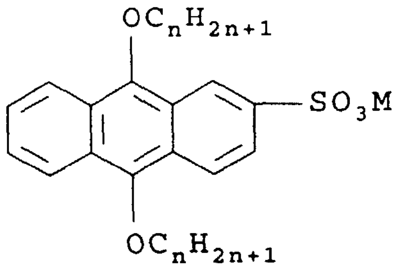

No. 28:

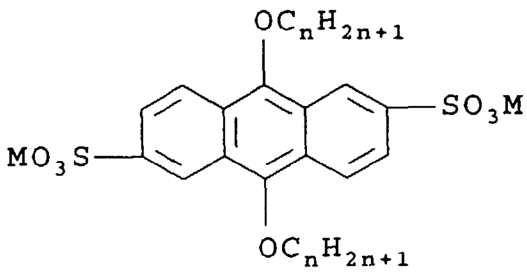

No. 29:

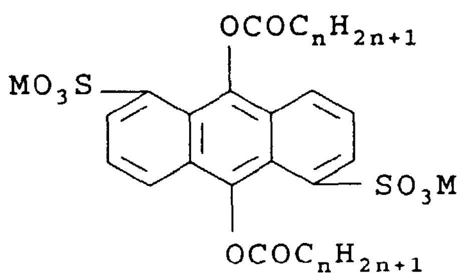

No. 30:

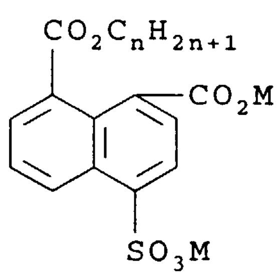

No. 31:

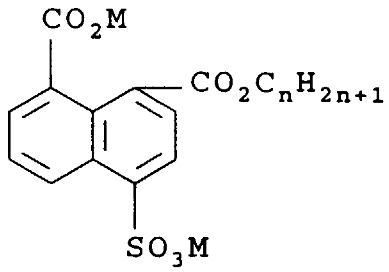

No. 32:

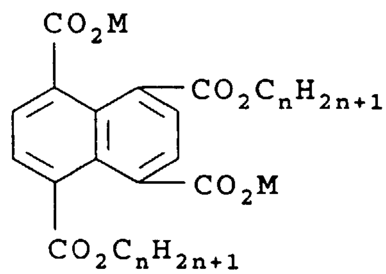

No. 33:

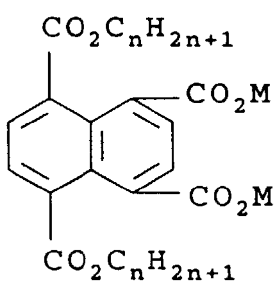

No. 34:

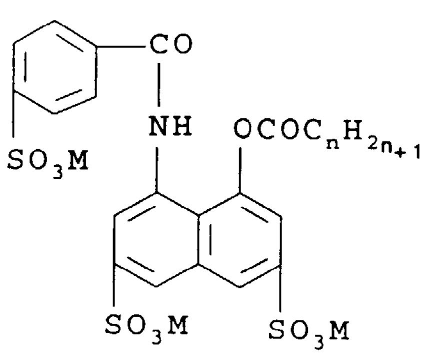

No. 35:

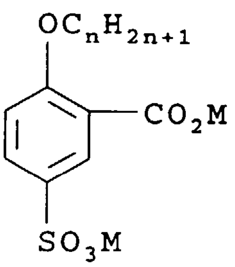

In structures 2 through 35 each n is between 1 to about 18, with the total number of alkyl carbon atoms preferably being between about 8 to about 30; M is H, or an alkali metal, pyridinium, or ammonium ion, preferably Na, K, or pyridinium.

In a preferred embodiment of the invention, a compound of Formula A is added to a gelatin containing photographic composition to thicken the composition when it is in the molten state. The resulting thickened melt is used to coat a layer of the composition onto a photographic support. The composition may be the only photographic layer coated on the support, but preferably is one of a plurality layers coated onto the support. More preferably, compositions of this invention are used for at least two of the individual layers of a multilayer photographic coating applied to a support.

Viscosity requirements for individual melts in multilayer coatings depend on the method and equipment (e.g. bead or curtain coatings) used for the coating process and upon many other considerations. Therefore, the ideal viscosity for any coating melt is in a small range that is most desirable for particular coating machine and melt composition. This invention provides a means for the enhancement of viscosity of a photographic melt to a given value that is ideal for a particular coating process and equipment. For this purpose, the lowest viscosity needed to be attained may lie anywhere between about 3 to 200 cP (mPa*s) at low shear rate (less than 100 $sec^{-1}$). A preferred range is above about 5 cP (mPa*s).

While in preferred embodiments of the invention the composition has a low gelatin content, it is to be understood that melts of photographic compositions of any desired gelatin content can be thickened in accordance with this invention.

As mentioned above, producing multilayer products using thickened photographic melts of this invention provide substantially defect free multilayer coatings. The term "substantially defect free multilayer coatings" means coatings that contain few, if any, observable or measurable layer thickness variations or other nonuniformity in the individual layers of the multilayer product coatings. The extent of nonuniformity which is acceptable depends on the type of photographic coating, the end product and its usage.

The coating melts of this invention are suitable mainly for multilayer coating machines using a multiple slide hopper or by the method of curtain coating.

The photographic products prepared in accordance with this invention may be a color paper product, color negative product, color transparency product, black-and-white paper, or film products, etc. as described in Research Disclosure, issue 308,119 pp. 993 to 1015, December 1989, the disclosure of which is incorporated herein by reference.

A preferred embodiment this invention is directed to a melt for the coating of a layer in a photographic element and which contains gelatin and an anionically charged, hydrophobic group containing compound that is (a) water soluble or rendered water soluble by solvent assistance, and (b) which confers a desirably high viscosity on gelatin melts.

It is preferred that the coating melts have a composition such as set forth in the following table:

| Coating Melts of the Invention | | |
|---|---|---|
| Component | Composition (weight percent) | |
| | Preferred | More Preferred |
| (a) gelatin | 3-20 | 3-15 |
| (b) silver halide emulsion | 0-50 | 0-40 |
| (c) coupler dispersion | 0-40 | 0-30 |
| (d) compound of Formula A | 0.1-20 | 0.1-10 |

The thickening agent (i.e., a compound of Formula A) that produce high viscosity in gelatin-containing melts, usually has the following criteria.

They comprise molecules with large hydrophobic groups solubilized by one or more fully charged anionic groups such as $-SO_3^-$ groups, sulfate groups or carboxy groups.

The water solubility characteristics range from fully water soluble, to solubility of at least about 5% in a blend of water and about 5% to about 20% by weight of a water miscible organic solvent such as methanol, ethanol, propanol, isopropanol, acetone, methyl ethyl ketone, ethyl acetate, etc. at temperatures between 35 to 50°C. (Use of such water miscible solvent in the amount specified is referred to herein as "solvent assisted" or "solvent enhanced" or "solvent enhancement".)

The thickening agent is usually added to melts containing gelatin and other photographic components from a clear aqueous or a mixed solution, such that the temperature of the mixture of the solution and the gelatin solution is between 35 to 50°C.

While not wishing to be bound by any theory, it is believed that there are a number of reasons why such photographic thickening agents produce higher melt viscosities when added to gelatin-containing photographic compositions. Figure 1 shows that gelatin molecules 10 have hydrophobic segments 12 (marked by a heavy line). The hydrophobic segments 12 of a plurality of gelatin molecules can attach to the hydrophobic areas of the charged thickening agents 14, thereby effectively increasing the molecular weight of the gelatin in the melt. Attachment of multiples of such thickening agent 14/gelatin 10 units to each other (as shown in Fig. 2) can produce substantially higher viscosities.

Gelatin is polypeptide with pendant carboxyl and various amine groups. At any given pH, depending upon the pKa of the amine groups, the gelatin molecule has both positive and negative charges (cf. T.H. James, The Theory of the Photographic Process, 4th Ed., MacMillan, New York, 1977). As shown in configuration 4 of Fig. 3, due to the partial neutralization of the various ionized groups, the gelatin molecule is fairly compactly coiled, especially so near the isoelectric point of the gelatin molecule. In configuration 4 of Fig. 3, the hydrophobic sections of the gelatin molecule are marked with heavier lines designated by 2. Negatively charged smaller hydrophobic molecules, such as those of the photographic agent 3 in question, may individually bind to the hydrophobic sites of the gelatin molecule and render it highly charged. In such a case, due to charge repulsion, the gelatin molecule will acquire a highly expanded structure as shown in configuration 6 of Fig. 3. This process of enhanced excluded volume will also lead to substantial enhancement of viscosity of the gelatin melt, and is an alternate mechanistic explanation for the viscosity enhancement process discussed earlier. It is to be understood that the effectiveness of the compounds added to photographic melts in accordance with this invention may be due undiscovered reasons.

The following examples are intended to be illustrative and not exhaustive illustrations of this invention. Parts and percentages are by weight unless otherwise specified.

<u>Preparative Example</u>

A coumpound of structure No. 1, above, for use as a thickening agent for photographic compositions in accordance with this invention was prepared according to the following reaction:

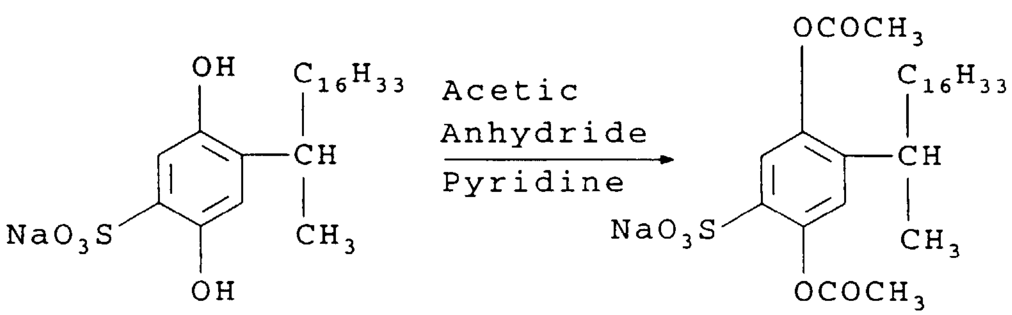


No. 36 No. 1

Acetic anhydride (25 mL) was added to a mixture of compound 36 in 25 mL of pyridine with stirring. The mixture was then heated at 80°C for 16 hours. After cooling to room temperature the reaction mixture was diluted with acetone (100 mL) and the product compound No. 1 was isolated by filtration as a white solid, m.p. 223-225°C. Yield was 7.9 g. Proton NMR and elemental analysis were consistent with the structure.

Compound 36 is an oxidized developer scavenger widely used in the photographic industry.

Compound 36 being an oxidized developer scavenger is not useful as a gelatin melt thickening agent as it produces photographic sensitometric changes. However, since the phenol groups are blocked in the compound 1, it is not expected to have any effect on photographic sensitometry.

Melt Examples 1 Through 6

Photographic melts containing very low concentrations of photographic gelatin (deionized lime processed ossein) were prepared. Each composition coantained 3.5% gelatin and compound 1 (prepared above) at levels between 0 and 20% by weight as indicated in Table 1. The pH of all the melts were adjusted to 7.0 using few drops of $10^{-3}$M NaOH solution.

Rheograms of melt Examples 1 through 6 were measured using a Rheometrics rheogeneometer at 40°C. and are shown in Fig 4. Rheograms of Fig. 4 depict the viscosities of these melts as a function of shear rate.

The gelatin melt of Example 1 with no additive of this invention, called the comparison example, has a very low viscosity of about 3 cP (mP*s) (at 100/sec shear rate) and it is virtually Newtonian as indicated by its virtual independence of shear rate. In Table I are listed viscosities of all the melt examples at 40°C. and a shear rate of 100/sec. The values of the slopes of the rheogram between 100 and 200/sec are also indicated in this table. The virtual zero slope of the rheogram of melt Example 1 is indicative of its Newtonian behavior.

Table I

| Composition and Rheology of Aqueous Gelatin Melts of This Invention | | | | | | |
|---|---|---|---|---|---|---|
| | Composition | | | | Rheology at 40°C. | |
| Example | % Gelatin | % Cmpd | % Water | pH | Viscosity n in cP or mP*s at 100/sec | Slope of Rheogram[1] of Fig. 4 |
| 1 (comparison) | 3.5 | 0.0 | 96.5 | 7.0 | 3.0 | 0.00 |
| 2 (invention) | 3.5 | 2.5 | 94.0 | 7.0 | 5.2 | 0.00 |
| 3 (invention) | 3.5 | 5.0 | 91.5 | 7.0 | 9.8 | -0.03 |
| 4 (invention) | 3.5 | 10.0 | 86.5 | 7.0 | 11.0 | -0.04 |
| 5 (invention) | 3.5 | 15.0 | 81.5 | 7.0 | 17.1 | -0.05 |
| 6 (invention) | 3.5 | 20.0 | 76.5 | 7.0 | 19.5 | -0.09 |

[1] Between shear rates of 100 and 200 $s^{-1}$.

It is seen in Table I and Figs. 4 and 5 that increasing the concentration of the thickening agent of structure No. 1 causes a drastic increase of the viscosity of the prepared melt. At 20% concentration of compound 1 a viscosity of 19.5 cP (mP*sec) is reached. This is about a 600% increase of viscosity of a gelatin melt containing 3.5% gelatin, indicating the extreme gelatin thickening activity of the thickening agent.

It should be noted that all the melts containing compound 1 show virtually no shear rate dependence of the melt viscosities (Fig. 4 and Table I). This is an added advantage of the invention as the melt thickening advantage will be realized even at very high coating speeds.

**Claims**

**1.** Use of a compound of the formula:

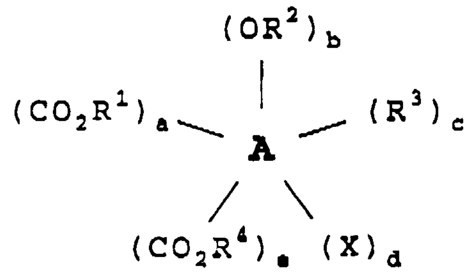

wherein:

A is an aromatic or heteroaromatic group;
X is $SO_3M$ or $CO_2M$, where M is H or an alkali metal, ammonium or pyridinium ion;
each $R^1$, $R^2$, and $R^3$ is independently an alkyl or substituted alkyl group, each alkyl group being straight or branched chain and having 1 to 30 carbon atoms;
$R^4$ is H or an alkyl or substituted alkyl group, each alkyl group being straight or branched chain and having 1 to 30 carbon atoms;
each of a, b, c and e is independently 0, 1, 2 or 3, with the proviso the sum of a + b + c + e equals at least 1; and d is 1, 2, or 3;

with the further proviso that the sum of a + b + c + e is at least 2 when A is phenyl, X is $SO_3M$, d is 1 and any one of a, c, or e is 1;

as a thickening agent in a photographic composition comprising an aqueous medium containing a hydrophilic colloid.

2. Use of claim 1 wherein A is phenyl, X is $SO_3M$, M is H, or a sodium, potassium, pyridium, or ammonium ion, $R^3$ is a straight or branched chain alkyl group having 1 to 30 carbon atoms, $R^4$ is a straight or branched chain alkyl group having 1 to 30 carbon atoms, d is 1, 2 or 3, c is 1, e is 2 or 3 and a and b are each 0.

3. Use of claim 1, wherein said compound has the formula:

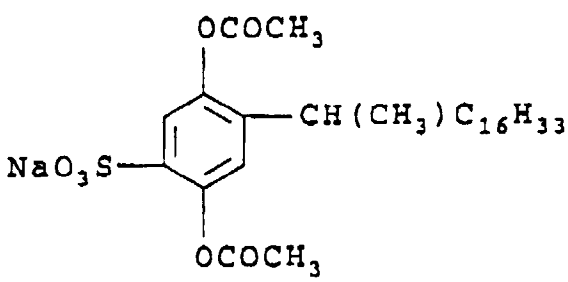

4. Use of any preceding claim, wherein said compound is present in an amount of 0.1 to 20% by weight, based on the weight of the composition.

5. Use of any preceding claim, wherein said hydrophilic colloid comprises gelatin.

6. Use of any preceding claim, wherein said hydrophilic colloid is present in an amount of 3 to 20% by weight, based on the weight of the composition.

7. A compound of the formula:

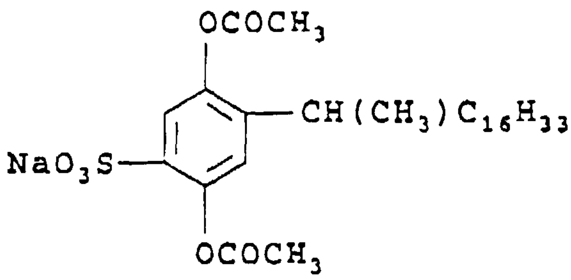

8. A photographic composition comprising the compound of claim 7.

**Patentansprüche**

1. Verwendung einer Verbindung der Formel:

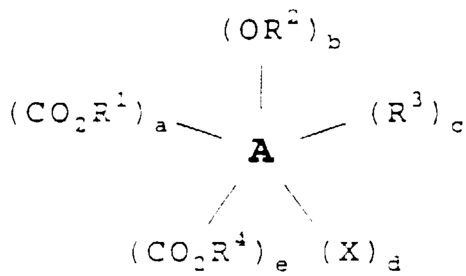

worin bedeuten:

A eine aromatische oder heteroaromatische Gruppe;

X gleich $SO_3M$ oder $CO_2M$, worin M für H oder ein Alkalimetall-, Ammonium- oder Pyridiniumion steht;

$R^1$, $R^2$ und $R^3$ jeweils unabhängig voneinander eine Alkyl- oder substituierte Alkylgruppe, wobei jede Alkylgruppe geradkettig oder verzweigtkettig ist und 1 bis 30 Kohlenstoffatome aufweist;

$R^4$ gleich H oder eine Alkyl- oder substituierte Alkylgruppe, wobei jede Alkylgruppe geradkettig oder verzweigtkettig ist und 1 bis 30 Kohlenstoffatome aufweist;

a, b, c und e jeweils unabhängig voneinander 0, 1, 2 oder 3, wobei gilt, daß die Summe von a + b + c + e mindestens gleich 1 ist; und

d gleich 1, 2 oder 3;

wobei weiterhin gilt, daß die Summe von a + b + c + e mindestens 2 beträgt, wenn a für Phenyl steht, X gleich $SO_3M$ ist, d gleich 1 ist und irgendeiner von a, c oder e für 1 steht;

als Dickungsmittel in einer photographischen Zusammensetzung mit einem wäßrigen Medium, das ein hydrophiles Kolloid enthält.

2. Verwendung nach Anspruch 1, worin A für Phenyl steht, X für $SO_3M$ steht, M gleich H ist oder ein Natrium-, Kalium-, Pyridium- oder Ammoniumion, worin $R^3$ für eine geradkettige oder verzweigtkettige Alkylgruppe mit 1 bis 30 Kohlenstoffatomen steht, $R^4$ eine geradkettige oder verzweigtkettige Alkylgruppe mit 1 bis 30 Kohlenstoffatomen ist, d für 1, 2 oder 3 steht, c gleich 1 ist, e gleich 2 oder 3 ist und a und b jeweils gleich 0 sind.

3. Verwendung nach Anspruch 1, wobei die Verbindung die Formel hat:

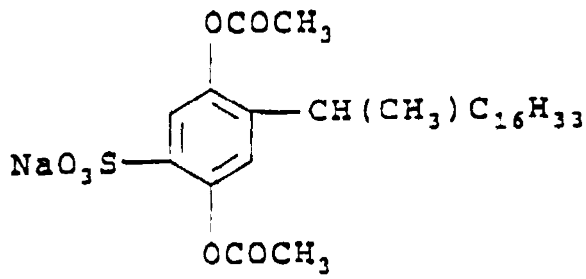

4. Verwendung nach einem der vorstehenden Ansprüche, wobei die Verbindung in einer Menge von 0,1 bis 20 Gew.-%, bezogen auf das Gewicht der Zusammensetzung, vorliegt.

5. Verwendung nach einem der vorstehenden Ansprüche, wobei das hydrophile Kolloid Gelatine umfaßt.

6. Verwendung nach einem der vorstehenden Ansprüche, wobei das hydrophile Kolloid in einer Menge von 3 bis 20 Gew.-%, bezogen auf das Gewicht der Zusammensetzung, vorliegt.

7. Verbindung der Formel:

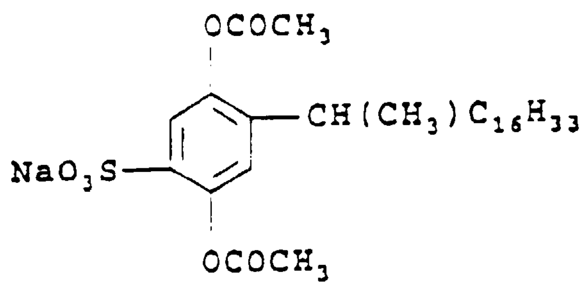

8. Photographische Zusammensetzung mit der Verbindung nach Anspruch 7.

**Revendications**

1. Utilisation d'un composé de formule :

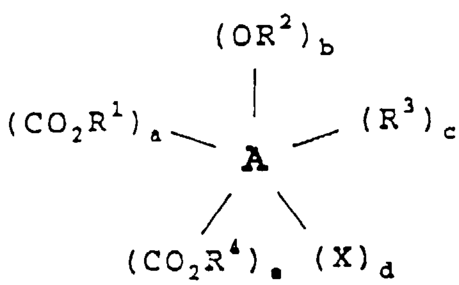

où :

A est un groupe aromatique ou hétéroaromatique ;
X est $SO_3M$ ou $CO_2M$, où M est H ou un métal alcalin, un ion ammonium ou pyridinium ;
chaque groupe $R^1$, $R^2$ et $R^3$ est indépendamment un groupe alkyle ou alkyle substitué, chaque groupe alkyle

étant à chaîne droite ou ramifiée et ayant de 1 à 30 atomes de carbone ;
$R^4$ est H ou un groupe alkyle ou alkyle substitué, chaque groupe alkyle étant à chaîne droite ou ramifiée et ayant de 1 à 30 atomes de carbone ;
a, b, c et e sont indépendamment 0, 1, 2 ou 3, à la condition que la somme de a + b + c + e soit égale à au moins 1 ; et
d est 1, 2 ou 3 ;
à la condition supplémentaire que la somme de a + b + c + e soit au moins égale à 2, lorsque A est phényle, X est $SO_3M$, d est 1 et l'un quelconque de a, c ou e est 1 ;
comme agent épaississant dans une composition photographique comprenant un milieu aqueux contenant un colloïde hydrophile.

2. Utilisation selon la revendication 1, dans lequel A est un groupe phényle, X est $SO_3M$, M est H ou un ion sodium, potassium, pyridium ou ammonium, $R^3$ est un groupe alkyle à chaîne droite ou ramifiée de 1 à 30 atomes de carbone, $R^4$ est un groupe alkyle à chaîne droite ou ramifiée de 1 à 30 atomes de carbone, d est 1, 2 ou 3, c est 1, e est 2 ou 3 et a et b sont chacun 0.

3. Utilisation de la revendication 1, dans laquelle ledit composé est représenté par la formule :

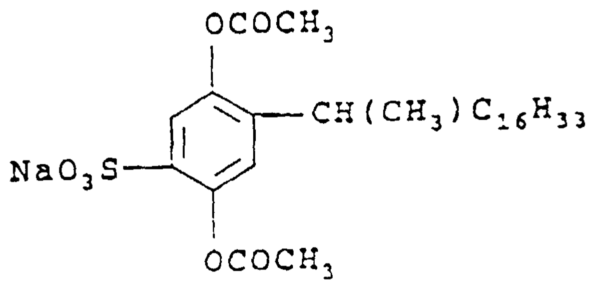

4. Utilisation de l'une quelconque des revendications précédentes, dans laquelle ledit composé est présent selon une quantité comprise entre 0,1 et 20 % en poids par rapport au poids de la composition.

5. Utilisation de l'une quelconque des revendications précédentes, dans laquelle ledit colloïde hydrophile comprend de la gélatine.

6. Utilisation de l'une quelconque des revendications précédentes, dans laquelle ledit colloïde hydrophile est présent selon une quantité comprise entre 3 et 20 % en poids par rapport au poids de la composition.

7. Composé de formule :

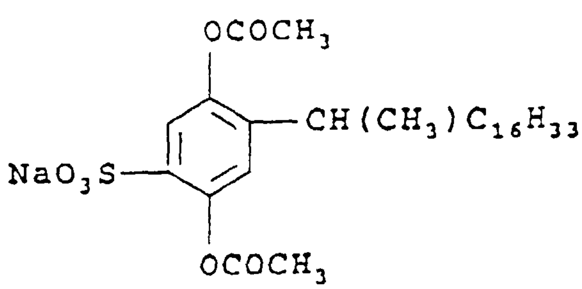

8. Composition photographique comprenant le composé selon la revendication 7.

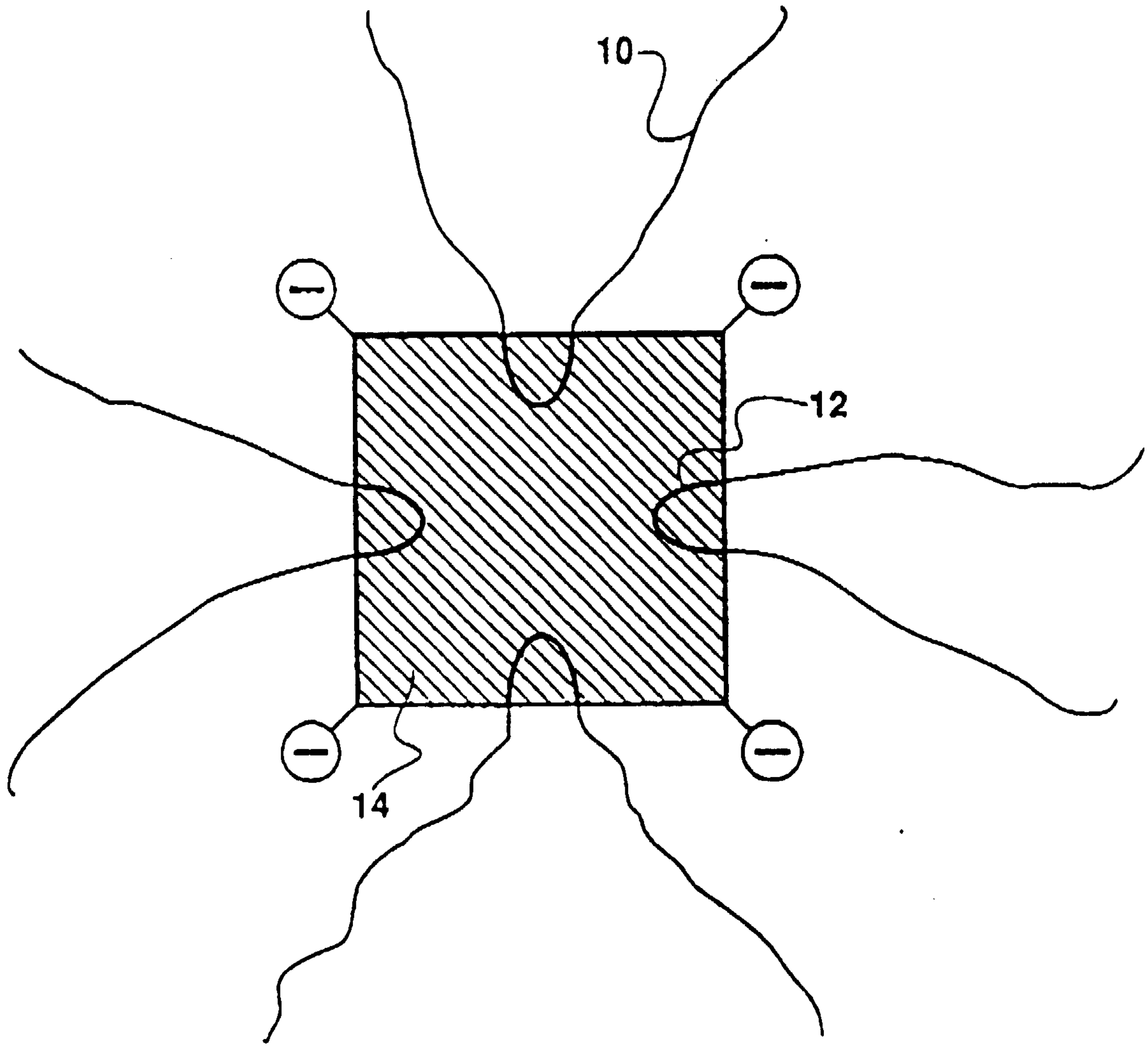


FIG. 1

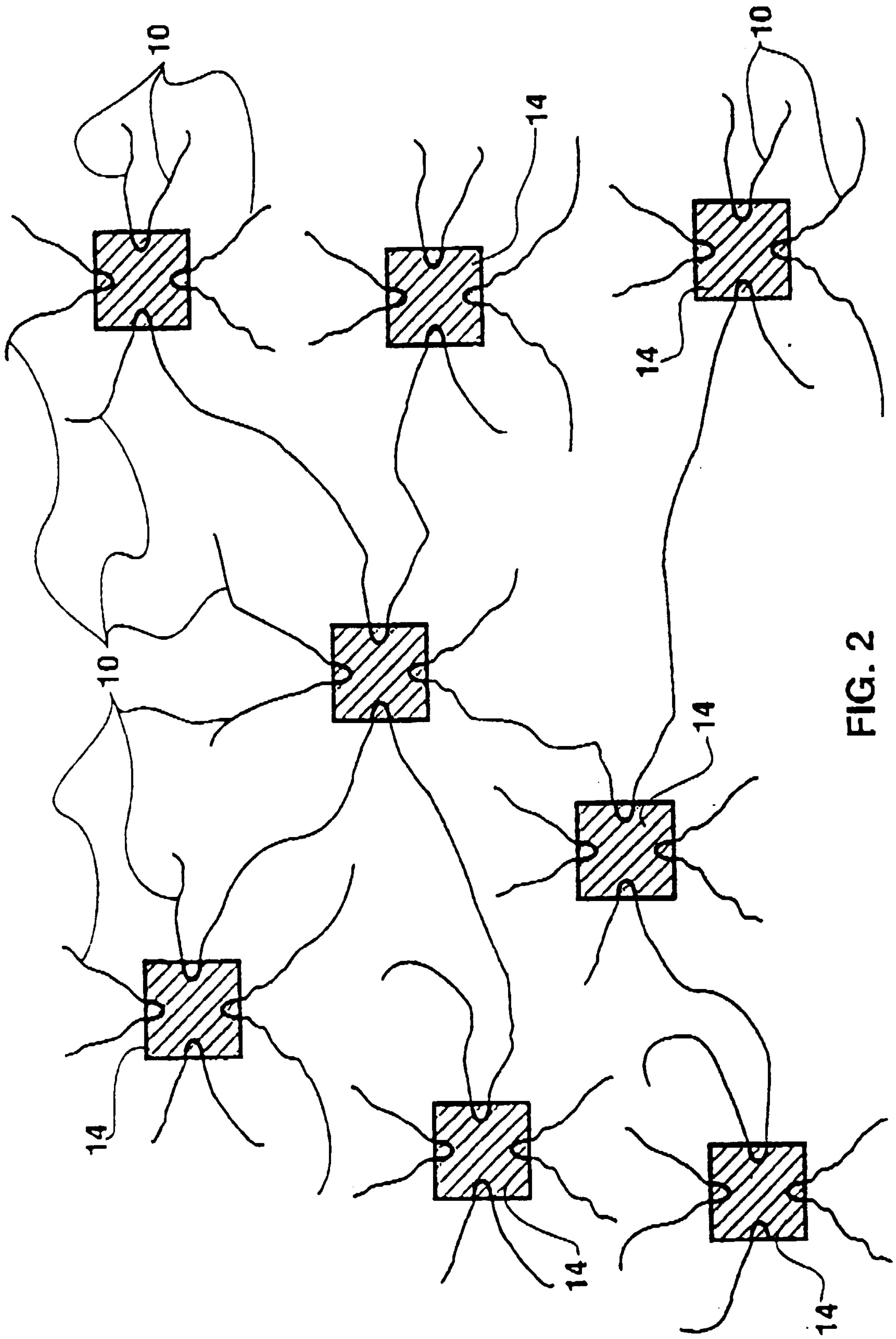


FIG. 2

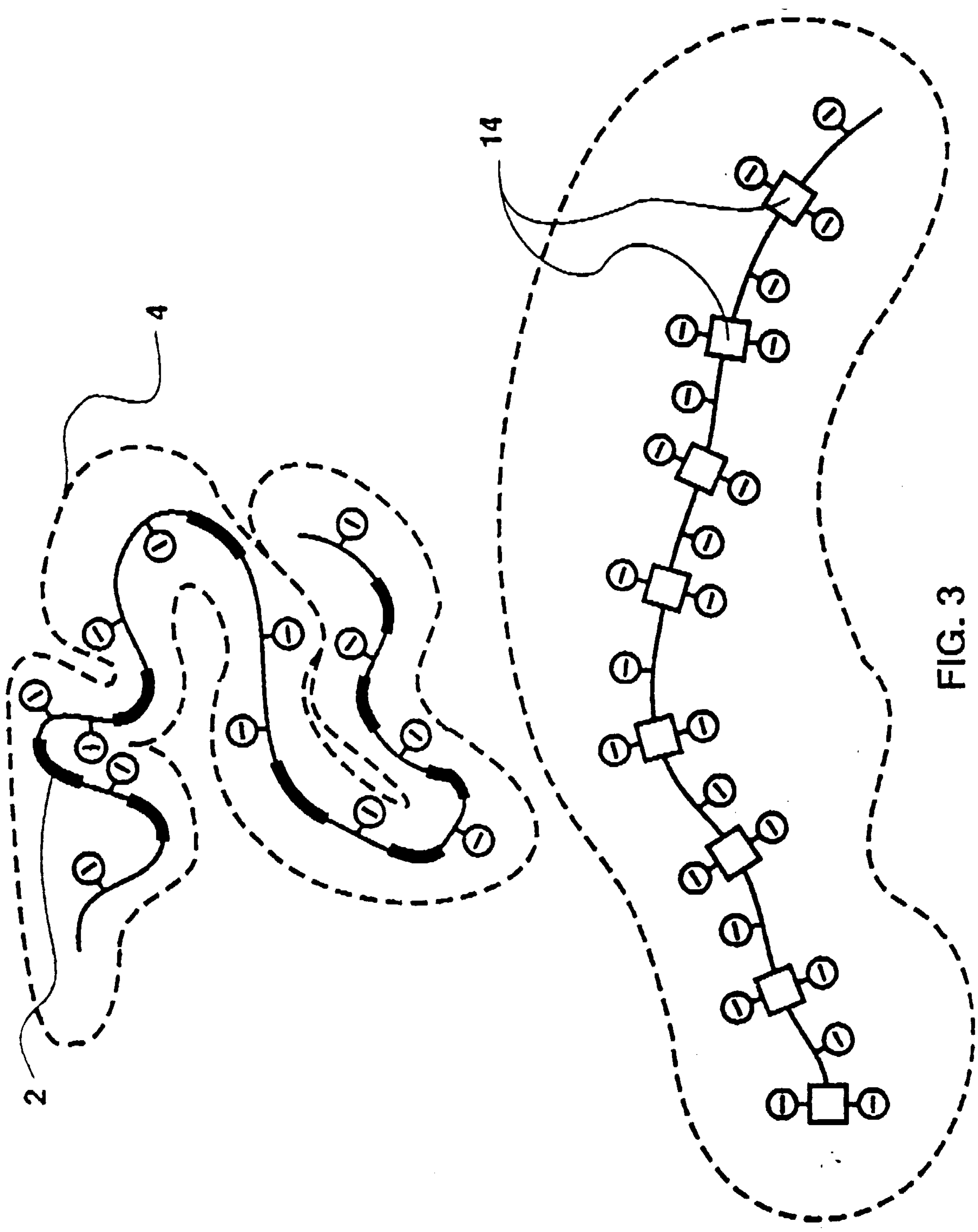


FIG. 3

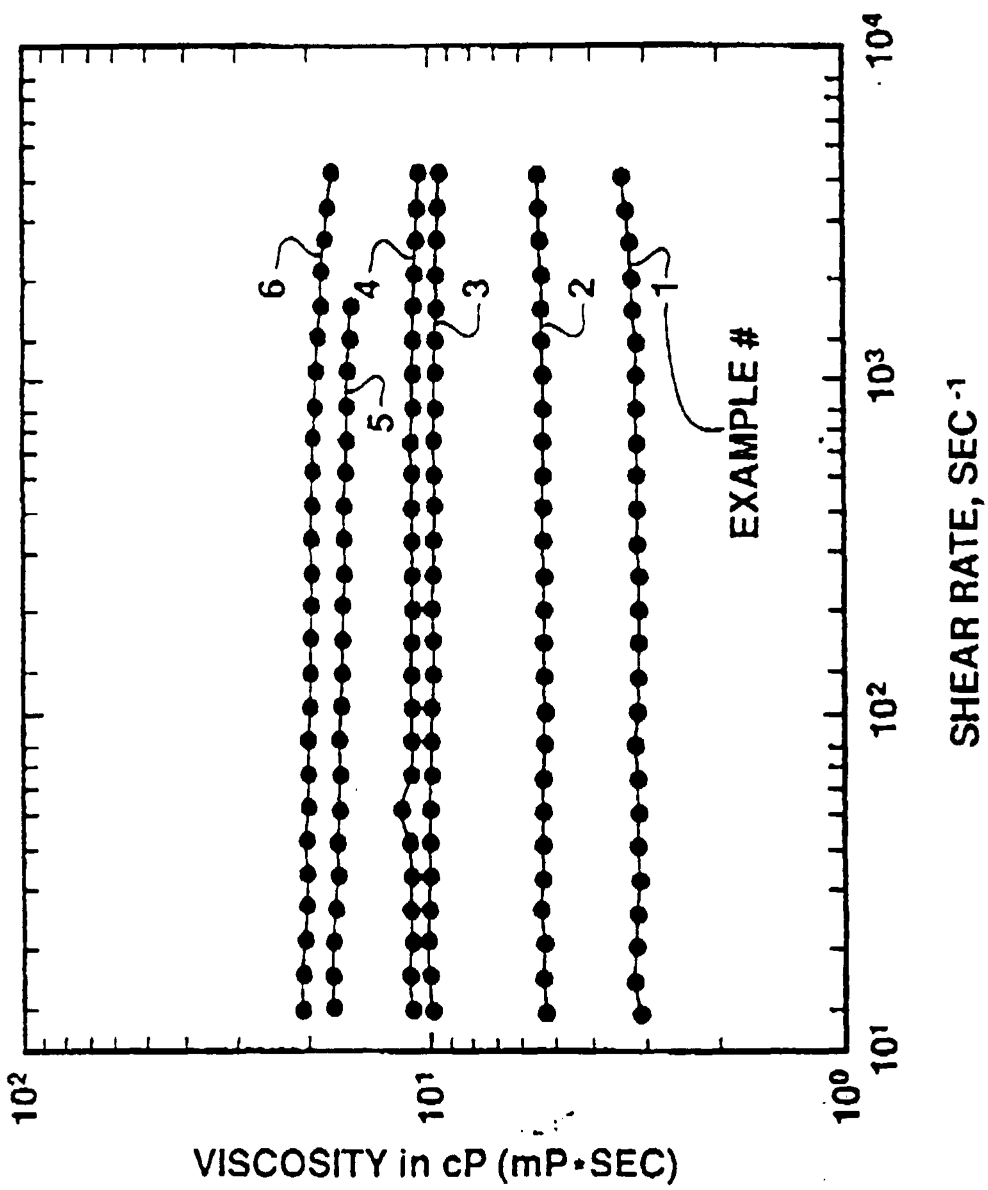
VISCOSITY in cP (mP*SEC)
$10^2$
$10^1$
$10^0$
SHEAR RATE, SEC$^{-1}$
$10^1$
$10^2$
$10^3$
$10^4$
EXAMPLE #
1
2
3
4
5
6

FIG. 4

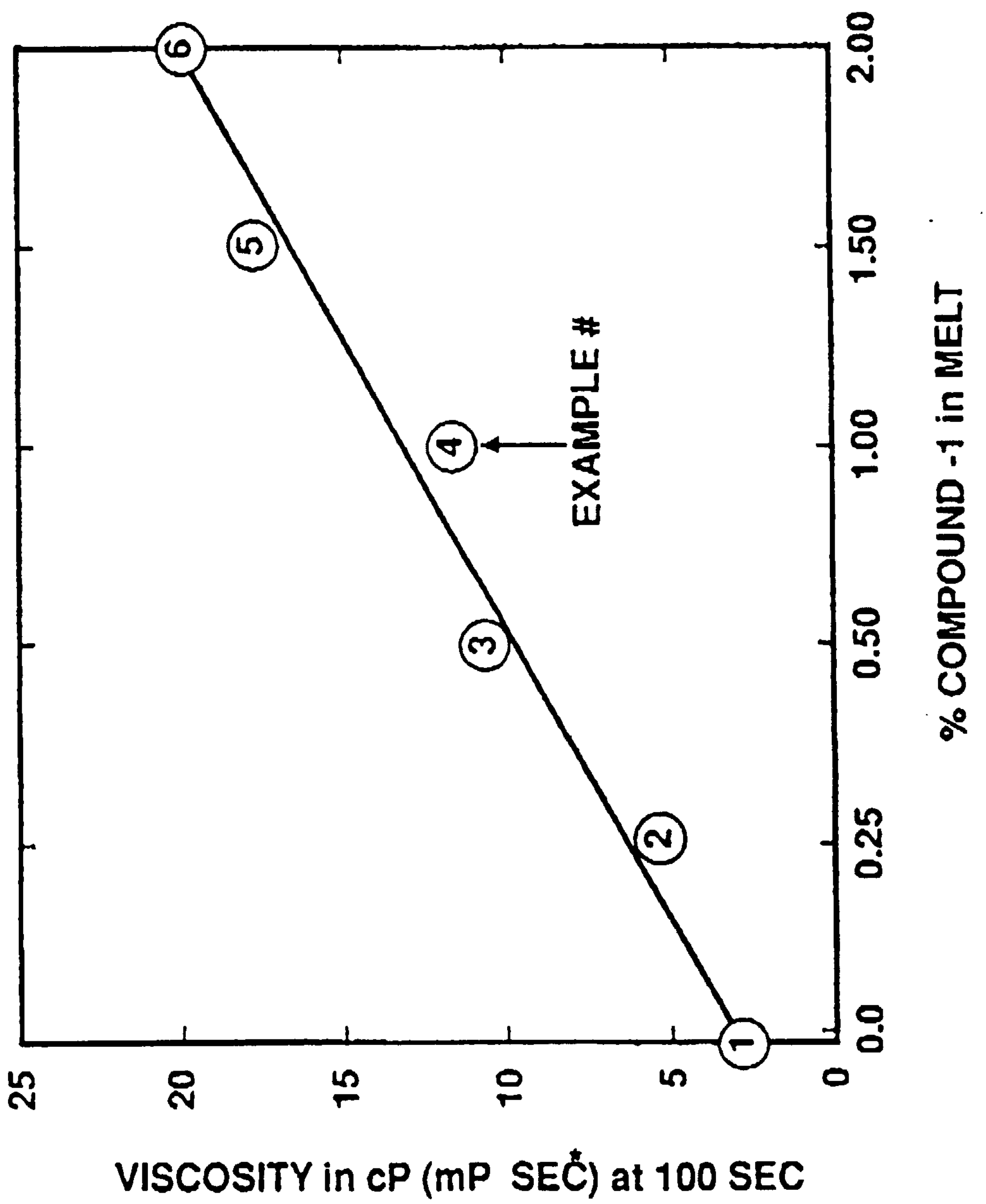
VISCOSITY in cP (mP SEC*) at 100 SEC
25
20
15
10
5
0
% COMPOUND -1 in MELT
0.0
0.25
0.50
1.00
1.50
2.00
EXAMPLE #
1
2
3
4
5
6

FIG. 5